# EUROPEAN PATENT APPLICATION

(11) **EP 2 589 355 A1**
(43) Date of publication of application: **08.05.2013**
(21) Application number: 11800139.5
(22) Date of filing: 21.06.2011
(51) Int. Cl.: A61F 5/442, A61G 9/02

(54) **EXCREMENT AUTOMATIC CLEANING APPARATUS WITHOUT TAKING IT OFF**

(30) Priority: 29.06.2010 CN 201010217657
(71) Applicant: Huang, Chunming, Nanan, Fujian 362000 (CN)
(72) Inventor: Huang, Chunming, Nanan, Fujian 362000 (CN)
(74) Representative: Chaillot, Geneviève
(86) International application number: PCT/CN2011/076003
(87) International publication number: WO 2012/000394

(57) **Abstract**

An excrement automatic cleaning apparatus without taking it off comprises a fully automatic disposal apparatus and a toilet. The toilet is provided with a waste collecting area (911), an excrement and urine discharging opening, a hot water input pipe and a hot air input pipe. The fully automatic disposal apparatus comprises a housing, an evacuation barrel (1), a negative pressure device (2), a hot water device (3), a hot air device (4) and a control device (6). The fully automatic disposal apparatus is further provided with a packing device (5) which is connected with the evacuation barrel (1) and can seal and pack the excrement, the urine and the waste water. The toilet is self-adapting pants (9) made of a soft facing material. The waste collecting area (911) is arranged in the crotch of the self-adapting pants and made of a soft and water-proof material. The fully automatic disposal apparatus is further provided with a positive pressure device (7) which can fill the self-adapting pants (9) with air so that the waste collecting area (911) is expanded to form a discharging space. The toilet can be worn on a user for a long time. The user does not need any help from others during the defecation, and the sanitary disposal after the defecation is simple.

## Description

### Technical Field

The present invention relates to the field of personal and household sanitation device, and more particularly to an excrement automatic cleaning apparatus without need of undressing and dressing which is designed for the mobility disabled person.

### Background Art

Currently put into the market is an excrement and urine automatic disposal system, which is designed for the mobility disabled person and serves to automatically dispose excrement and urine of the mobility disabled person, and which generally comprises an excrement and urine automatic disposal apparatus and a toilet cooperating with the excrement and urine automatic disposal apparatus. The excrement and urine automatic disposal apparatus is generally provided with an evacuation barrel, a negative pressure device, a hot water device, a hot air device and a control device. The evacuation barrel accommodates excrement and urine discharged from the human body. The toilet is generally provided with an excrement collecting area, an excrement and urine discharging port, a hot water inlet pipe and a hot air inlet pipe. The excrement collecting area is further provided with an excrement and urine discharging port which is connected with an excrement and urine discharging duct. The evacuation barrel is connected with the excrement and urine discharging duct via the negative pressure device, for drawing the excrement and urine into the evacuation barrel by negative pressure. The hot water inlet pipe is connected with the hot water device via a hot water pipe, for flushing the excrement and urine place of the user. The hot air inlet pipe is connected with the hot air device via a hot air pipe, for drying the excrement and urine place of the user. The control device further comprises an interactive interface which is provided with an ON/OFF switch. In particular, the control device may further be provided with a remote controller for switching ON/OFF.

However, in the existing excrement and urine automatic disposal apparatus, once the mobility disabled person completes excrement and urine, a relatively large amount of hot water is required for washing, the moisture volatilizes relatively slowly, and it consumes more energy. In addition, at predetermined time or when the excrement and urine container reaches a certain capacity, it is generally necessary to remove the excrement and urine container from the disposal apparatus, dump in the bathroom, and flush with water. In this process, the caregiver may catch sight of the excrement and urine with eyes, smell the excrement and urine with noses, and wash the evacuation barrel manually with cleaning tools.

In addition, the existing toilets for cooperating with the excrement and urine automatic disposal apparatus have a common feature in that, when the mobility disabled person needs defecation, it is necessary for the user to have a self-care ability or a caregiver to install various toilets as mentioned above to the excrement and urine discharging place; and when the defecation is complete, it is also necessary for the caregiver to remove the toilets. It is impossible to automatically and intelligently complete the process of discharging the excrement and urine as well as sanitary disposal after the defecation without help from others. Especially due to the operation mode, material and structure of the existing toilet, it can not keep contact with the human body for a long time, and can only be removed from the excrement and urine discharging place by means of a mechanical transmission means or manually by the caregiver. It is relatively inconvenient for use.

In view of this, the inventors have studied the structure of the existing excrement and urine automatic disposal system in depth, and thereby accomplished the present application.

### Disclosure of the Invention

It is an object of the present invention to provide an excrement automatic cleaning apparatus without need of undressing and dressing. The toilet can be worn on a user for a long time. The user does not need any help from others during defecation, and the sanitary disposal after the defecation is simple. The advantages of lie in that there is no need of undressing and dressing the toilet and it is convenient, simple and comfortable to use.

The following solutions are proposed in the present invention to achieve the above objects.

An excrement automatic cleaning apparatus without need of undressing and dressing comprises a fully automatic disposal apparatus and a toilet. The toilet is provided with an excrement collecting area, an excrement and urine discharging port, a hot water inlet pipe and a hot air inlet pipe, which cooperate with the fully automatic disposal apparatus. The fully automatic disposal apparatus comprises a housing, and an evacuation barrel, a negative pressure device, a hot water device, a hot air device and a control device in the housing. The evacuation barrel is connected with the excrement and urine discharging port of the toilet via the excrement and urine discharging duct. The control device is connected with the negative pressure device, the hot water device and the hot air device, respectively. The negative pressure device, the hot water device and the hot air device are connected with the evacuation barrel, the hot water inlet pipe and the hot air inlet pipe, respectively. The fully automatic disposal apparatus is further provided with a packing device which is connected with the evacuation barrel and can seal and pack the excrement, urine and waste water. The toilet is self-adapting pants made of a soft facing material. The excrement collecting area is arranged at the crotch of the self-adapting pants and made of a soft and water-proof material. The fully automatic disposal apparatus is further provided with a positive pressure device which can inflate the self-adapting pants so that the excrement collecting area of the self-adapting pants is expanded to form a discharging space.

The housing comprises a rear chamber and a front chamber which are arranged in a high/low manner. The evacuation barrel, the packing device and the control device are arranged in the rear chamber. The evacuation barrel and the packing device are arranged in an up/down manner. The control device is arranged at a side of the evacuation barrel, the front chamber contains a negative pressure device, a hot air device and a hot water device which are arranged sequentially from left to right. The outlets of an inlet pipe, a hot air output pipe and a hot water output pipe, which correspond to the evacuation barrel, the hot air device and the hot water device, extend from the same neighboring position of the housing.

The evacuation barrel is provided with an inlet pipe, a negative pressure pipe and an outlet pipe for communicating with the excrement and urine discharging duct, the negative pressure device and the packing device, the outlet pipe is arranged at the bottom of the evacuation barrel, the outlets of the inlet pipe and the negative pressure pipe are arranged in a staggered manner, and the outlet of the negative pressure pipe is higher than the outlet of the inlet pipe.

The evacuation barrel is further provided with an evacuation barrel control valve at the outlet pipe, and the evacuation barrel control valve is connected with the control device.

The evacuation barrel is provided with a hot water pipe for communicating with the hot water device, and the outlet of the hot water pipe is located above the inlet pipe.

The packing device comprises a packing bag and a packing bag accommodating case for accommodating the packing bag, the packing bag accommodating case is located below the evacuation barrel, the packing bag is provided with a mouth for connecting with the evacuation barrel, and the mouth is provided with a tightening belt for sealing this mouth.

The hot water device comprises a water tank and a water heating device which are arranged in an up/down manner, the water tank is provided with a water inlet for feeding water at the upper portion, and the water tank is provided with supporting bars for supporting the water tank at the bottom.

The control device comprises a primary controller, a data acquisition module connected with an input terminal of the primary controller and a driver output module connected with an output terminal of the primary controller.

The control device further comprises an interactive unit connected with the primary controller, which comprises a liquid crystal display module and a key input module.

The housing is provided with road wheels for facilitating the housing to move around at the bottom.

With the above-mentioned solutions, the excrement automatic cleaning apparatus without need of undressing and dressing the present invention has the following beneficial effects, as compared with the existing excrement and urine automatic disposal system.

Firstly, after the excrement and urine is flushed to the evacuation barrel, the excrement, urine and waste water in the evacuation barrel can be sealed and packed into a packing bag by the packing device. The caregiver only needs to throw the packed waste into a dustbin, and does not have to directly contact the excrement and urine, which can largely improve working conditions of the caregiver and facilitate carrying out the nursing services.

Secondly, by using self-adapting pants made of a soft facing material as the toilet, and designing appropriately the self-adapting pants, such a toilet in the form of pants not only has various functions of the existing toilet, but also is soft and comfortable, so that it can be worn on a user for a long time, and substantially make no difference from the ordinary pants in comfort, wearing process and changing time. During excrement and urine, the user does not need help from others to dress and undress the toilet, and can start the excrement disposal apparatus at any time by himself to defecate, so that it is very convenient for use.

Thirdly, the self-adapting pants are further provided with a positive pressure device, which can inflate the self-adapting pants, so that the excrement collecting area of the self-adapting pants is expanded to form a discharging space. As a result, the negative pressure device can smoothly draw the waste into the evacuation barrel, which also improves the comfort of the user during a defecation process.

### Brief Description of Drawings

Fig. 1 is a structural view showing the fully automatic disposal apparatus of the present invention;
Fig. 2 is a front view showing the internal structure of the fully automatic disposal apparatus of the present invention;
Fig. 3 is a rear view showing the internal structure of the fully automatic disposal apparatus of the present invention;
Fig. 4 is a structural view showing an evacuation barrel of the present invention;
Fig. 5 is a structural view showing a packing device of the present invention;
Fig. 6 is a structural view showing a water tank and a water heating device for a hot water device of the present invention;
Fig. 7 is a schematic view showing the structural principle of a positive pressure device of the present invention;
Fig. 8 is a view showing the control principle of a control device of the present invention;
Fig. 9 is a schematic view showing a process for disposing excrement and urine which requires flushing of the present invention;
Fig. 10 is a schematic view showing a process for disposing urine which does not require flushing of the present invention; and
Fig. 11 is a structural view showing a specific embodiment for self-adapting pants of the present invention.

### Best Mode for Carrying out the Invention

To further elucidate the solutions of the present invention, the present invention will hereinafter be set forth in detail by reference to the specific embodiments.

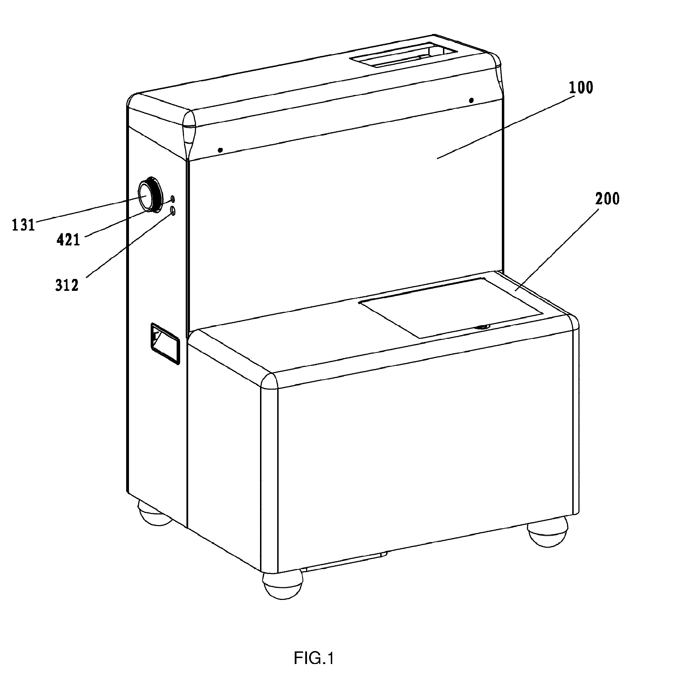

As shown in Figs. 1-11, an excrement automatic cleaning apparatus without need of undressing and dressing of the present invention comprises a fully automatic disposal apparatus and self-adapting pants 9. The self-adapting pants 9 are made of a soft facing material. The self-adapting pants 9 are provided with an excrement collecting area 911, an excrement and urine discharging port, a hot water inlet pipe and a hot air inlet pipe compatible with the fully automatic disposal apparatus. The excrement collecting area 911 is arranged at the crotch of the self-adapting pants 9, which is made of a soft and water-proof material.

The fully automatic disposal apparatus comprises a housing, as well as an evacuation barrel 1, a negative pressure device 2, a hot water device 3, a hot air device 4, a packing device 5, a positive pressure device 7 and a control device 6 in the housing.

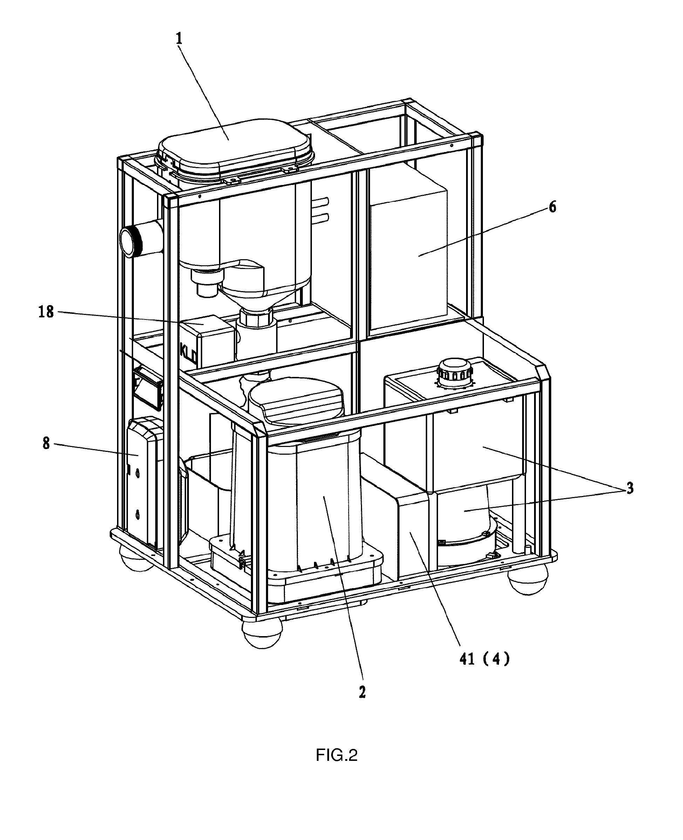

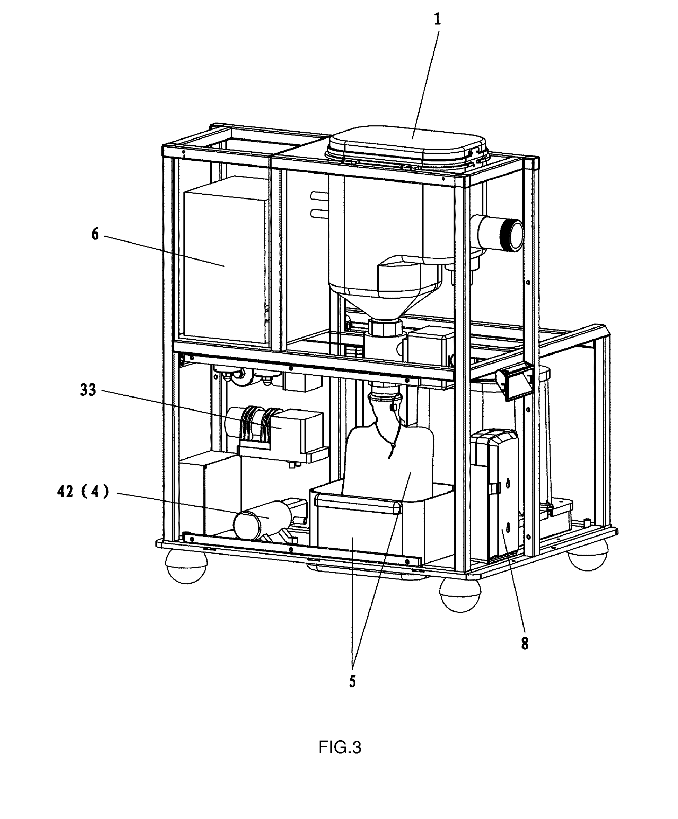

The housing comprises a rear chamber 100 and a front chamber 200 which are arranged in a high/low manner. The evacuation barrel 1, the packing device 5 and the control device 6 are arranged in the rear chamber 100. As shown in Fig. 3, in particular, the rear chamber 100 is divided into a left chamber and a right chamber. The evacuation barrel 1 and the packing device 5, which are arranged in an up/down manner, are provided in the right chamber of the rear chamber 100, and the control device 6 is provided in the upper portion of the left chamber of the rear chamber 100. As shown in Fig. 2, the front chamber 200 contains the negative pressure device 2, the hot air device 4 and the hot water device 3 which are arranged sequentially from right to left. The inlet 131 corresponding to the inlet pipe 13 of the evacuation barrel 1, and the outlets 421, 312 for the hot air output pipe and the hot water output pipe of the hot air device 4 and hot water device 3 are arranged adjacent to each other at the same position. In particular, the inlet and outlets extend from the side wall of the upper portion of the rear chamber 100. All of the devices are arranged in the housing orderly and compactly. In order to facilitate the fully automatic disposal apparatus to freely move around, the housing is provided at the bottom with road wheels for facilitating the housing to move around.

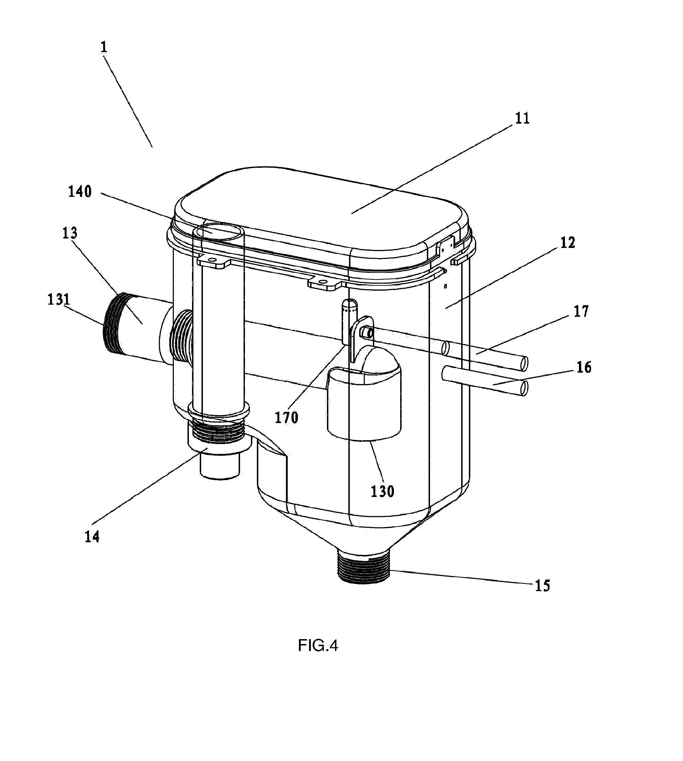

The evacuation barrel 1, as shown in Fig. 4, comprises a barrel top cover 11 and a barrel body 12. The barrel body 12 is provided with the inlet pipe 13. The inlet 131 of the inlet pipe 13 is connected with the excrement and urine discharging port of the self-adapting pants 9 via the excrement and urine discharging duct 92, so that the excrement and urine discharged by the human body sequentially passes through the excrement and urine discharging port, the excrement and urine discharging duct 92 and the inlet pipe 13, and finally enters the evacuation barrel 1. The barrel body 12 is provided with a negative pressure pipe 14 communicating with the negative pressure device 2 described later. The barrel body 12 is further provided at the bottom with an outlet pipe 15. A portion of the barrel body 12 at which the outlet pipe 15 is connected forms a funnel structure. The outlet 130 of the inlet pipe 13 is directed downwards to the funnel structure, so as to facilitate discharging the excrement and urine.

A vacuum pump in the negative pressure device 2 is connected with the negative pressure pipe 14 in the evacuation barrel 1, so that a negative pressure is produced in the evacuation barrel 1 to draw the excrement and urine discharged by the human body from the inlet pipe 13 into the evacuation barrel 1. In order to avoid the excrement and urine in the inlet pipe 13 is directly drawn into the negative pressure device 2, the outlets of the inlet pipe 13 and the negative pressure pipe 14 are arranged in a staggered manner, and the outlet of the negative pressure pipe 14 is located at a position higher than that of the outlet of the inlet pipe 13. As shown in Fig. 3, the negative pressure pipe 14 is arranged by inserting vertically into the barrel body 12 from the bottom, and the outlet 140 is slightly lower that the periphery of the opening of the barrel body 12. The inlet pipe 13 is arranged by laterally inserting from a side of the barrel body 12 and vertically bending downwards to confront the outlet pipe 15. The outlet 130 of the inlet pipe 13 and the outlet 140 of the negative pressure pipe 14 are arranged in a staggered manner, and the outlet 140 is higher than the outlet 130, thus ensuring smooth discharge of the excrement and urine. A pipe 16 is further arranged between the negative pressure device 2 and the evacuation barrel 1. The pipe 16 serves to remove moisture which is possibly present in the negative pressure device 2, so as to ensure the cleanliness and proper operation of the negative pressure device 2.

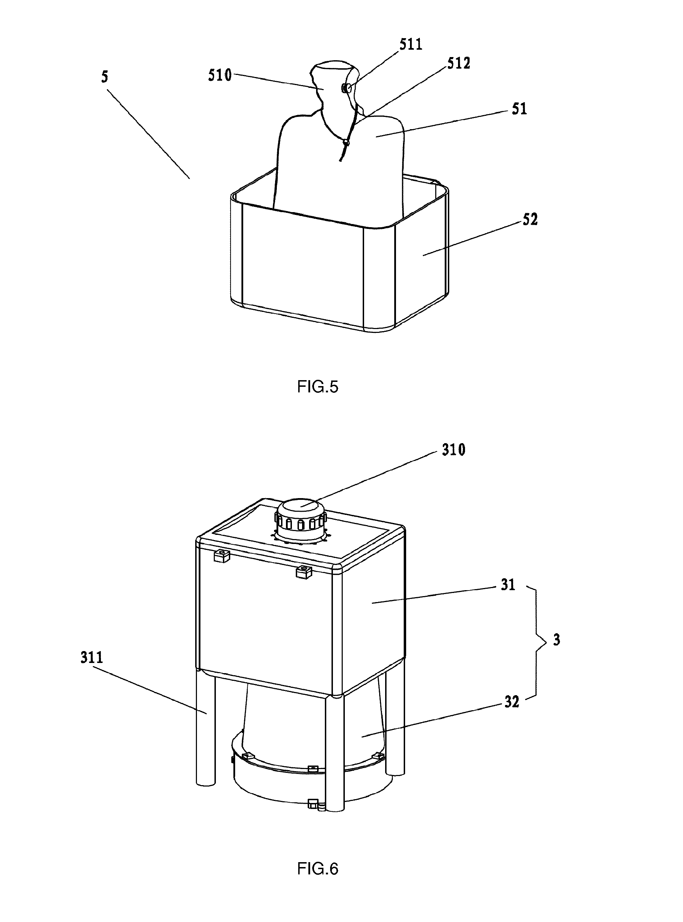

The packing device 5, as shown in Fig. 5, comprises a packing bag 51 and a packing bag accommodating case 52 for accommodating the packing bag 51. The packing bag accommodating case 52 is located below the evacuation barrel 1, and the packing bag 51 is provided with a mouth 510. As shown in Fig. 3, a connecting pipe is provided at the lower end of the outlet pipe 15, a mouth 510 is fastened to the connecting pipe, and a male/female buckle 511 is used to tightly fasten the mouth 510 to the connecting pipe. A flange is formed along the periphery of lower end of the connecting pipe. The male/female buckle 511 is located at the upper side of the flange, thus improving the strength of connection between the mouth 510 and the connecting pipe. A tightening belt 512 is further provided near the lower end of the mouth 510 of the packing bag 51. The tightening belt 512 can seal and pack the excrement, urine and waste water entering the packing bag 51. Besides, the outlet pipe 15 is further provided with an evacuation barrel control valve 18. The evacuation barrel control valve 18 is connected with the control device 6 described later, so as to discharge waste in the evacuation barrel 1 into the packing bag 51.

The hot water device 3 is connected with the hot water inlet pipe of the self-adapting pants 9 via the hot water output pipe. The water flow for flushing the user's excrement and urine place can be produced in the hot water device 3. After the water flow from the hot water device 3 flushes the user's excrement and urine place, it is directly passed by the inlet pipe 13 into the evacuation barrel 1 via the excrement and urine discharging duct 92 between the evacuation barrel 1 and the self-adapting pants 9. In particular, the hot water device 3 comprises a water tank 31, a water heating device 32 and a hot water pump 33 which are arranged in an up/down manner. As shown in Fig. 6, the upper portion of the water tank 31 is provided with a water inlet for feeding water and a cover 310 compatible with the water inlet. By unscrewing the cover 310, it is possible to feed water to the water tank 31. The water tank 31 is provided at the bottom with several supporting bars 311 for supporting the water tank 31. The water heating device 32 is located in a space formed by these supporting bars 311, and heat water in the water tank 31 from bottom. The water tank 31 is provided with a temperature sensor and a liquid level sensor which are connected with the control device 6, for detecting temperature and liquid level of water in the water tank 31. The water tank 31 is connected with the self-adapting pants 9 via the hot water pump 33. A certain water flow pressure is provided by the hot water pump 33 to flush the human body. In order to keep the evacuation barrel 1 clean, the hot water device 3 further comprises a flushing reversal valve. The flushing reversal valve is arranged in a hot water inlet pipe connected with the self-adapting pants 9. The flushing reversal valve is further connected with a hot water pipe 17 of the evacuation barrel 1 via a pipe. An outlet 170 of the hot water pipe 17 is located above the inlet pipe 13. In this way, by means of the flushing reversal valve, it is possible to flush efficiently the evacuation barrel 1, so as to improve the cleanliness and avoid bacteria growth.

The hot air device 4 comprises an air heating device 41 and a hot air pump 42. The hot air pump 42 is connected with the hot water inlet pipe of the self-adapting pants 9 via a hot air output pipe. By means of the pressure provided by the hot air pump 41, an air flow is produced in the hot air output pipe and the hot water inlet pipe for drying the user's excrement and urine place. After drying the user's excrement and urine place, the air flow is discharged via the negative pressure device 2.

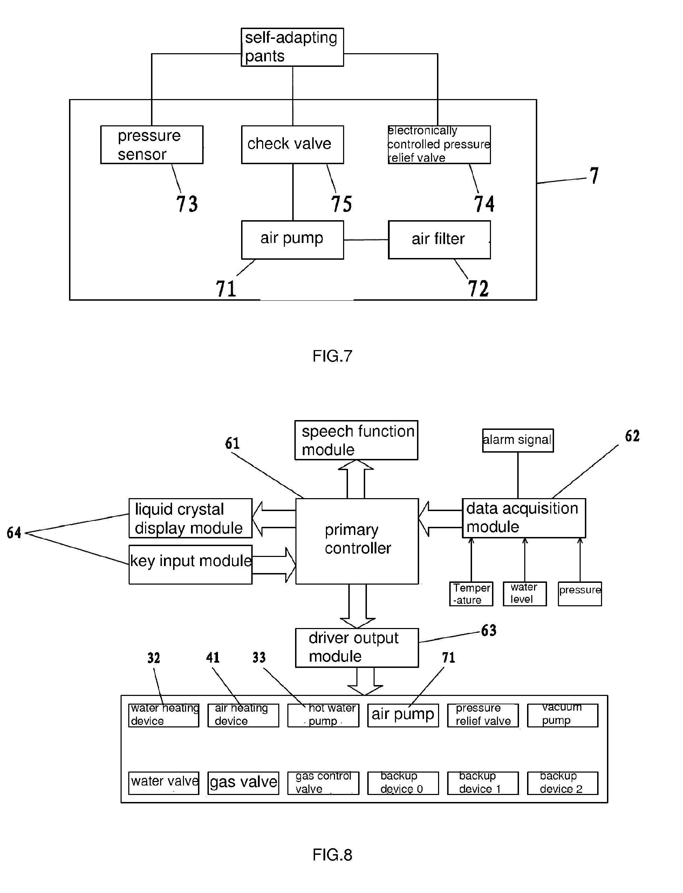

As shown in Fig. 7, the positive pressure device 7 is connected with respective pipe of the self-adapting pants 9 via the positive pressure output pipe. The positive pressure device 7 can inflate the self-adapting pants 9, so that the excrement collecting area of the self-adapting pants 9 is expanded to form a discharging space. As a result, the negative pressure device 2 can draw waste more smoothly into the evacuation barrel 1. As a preferred embodiment of the positive pressure device 7, the positive pressure device 7 comprises an air pump 71, an air filter 72, a pressure sensor 73, an electronically controlled pressure relief valve 74 and a check valve 75. The air pump 71 communicates with the atmosphere via the air filter 72, thus ensuring the cleanliness of the air. The check valve 75 is arranged in a pipe between the air pump 71 and the self-adapting pants 9, to maintain air pressure in the self-adapting pants 9. The electronically controlled pressure relief valve 74 and the pressure sensor 73 are both arranged in the self-adapting pants 9. The electronically controlled pressure relief valve 74 is connected with and controlled by the control device 6. Once the use of the human body excrement disposal apparatus is complete, the electronically controlled pressure relief valve 74 releases the air pressure in the self-adapting pants 9. The pressure sensor 73 functions as an overpressure protection.

The control device 6 is connected with the negative pressure device 2, the hot water device 3, the hot air device 4 and the positive pressure device 7, and controls the cooperation among them, i.e., controls the operating order of these devices. In particular, as shown in Fig. 8, the control device 6 mainly comprises a primary controller 61, a data acquisition module 62, a driver output module 63, an interactive unit 64 and a power supply module. The power supply module supplies power to the whole control device 6. The data acquisition module 62 can transfer the acquired data to the primary controller 61. By means of the reading, analyzing and controlling actions of the primary controller 61, the related control instructions are transferred to the driver output module 63. The driver output module 63 is connected with various devices, especially with the valves and bumps in these devices, to achieve related operations of the devices.

The data acquisition module 62 may be divided into temperature, water level and pressure sampling circuit units. The temperature sampling comprises sampling the temperature of water in the hot water device 3 and sampling the temperature of air flow in the hot air device 4, the water level sampling comprises sampling level of water in the water tank 31 of the hot water device 3, and the pressure sampling comprises negative pressure sampling in the negative pressure device 2 and positive pressure sampling in the positive pressure device 7. The data acquisition module 62 may further comprise an alarm unit, which issues an alarm signal to correct the user's operation, so as to improve the safety performance of the apparatus.

The interactive unit 64 is connected with the primary control module 61, and especially comprises a key input module and a liquid crystal display module, which may enable to set and modify the apparatus control parameters, set the operation mode, display the current operation status, and provide manual control buttons or the like. The interactive unit 64 may further use a LCD display with a touch screen, and may be further provided with a speech function module. In addition, it is also possible to provide a remote control unit, to start and stop the whole excrement automatic cleaning apparatus wirelessly.

It is inevitable that there is excrement smell in the fully automatic disposal apparatus. In order to reduce such smell and improve cleanliness of air, according to the present invention, the fully automatic disposal apparatus is further provided with an air refreshing device 8 which is connected with the control device 6. The air refreshing device not only refreshes the air in the cleaning apparatus, but also serves to sterilize the air within the cleaning apparatus.

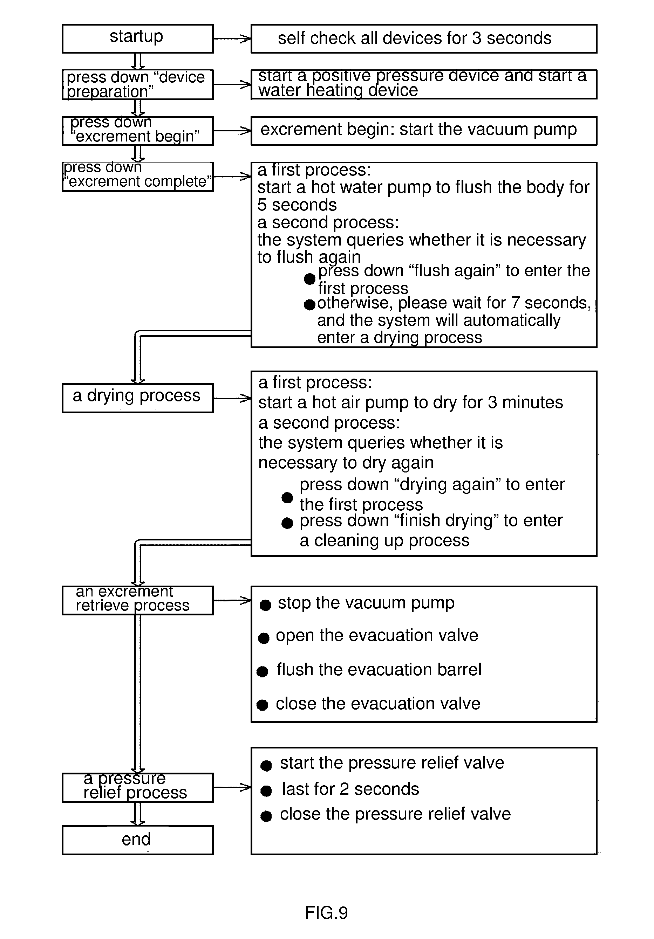

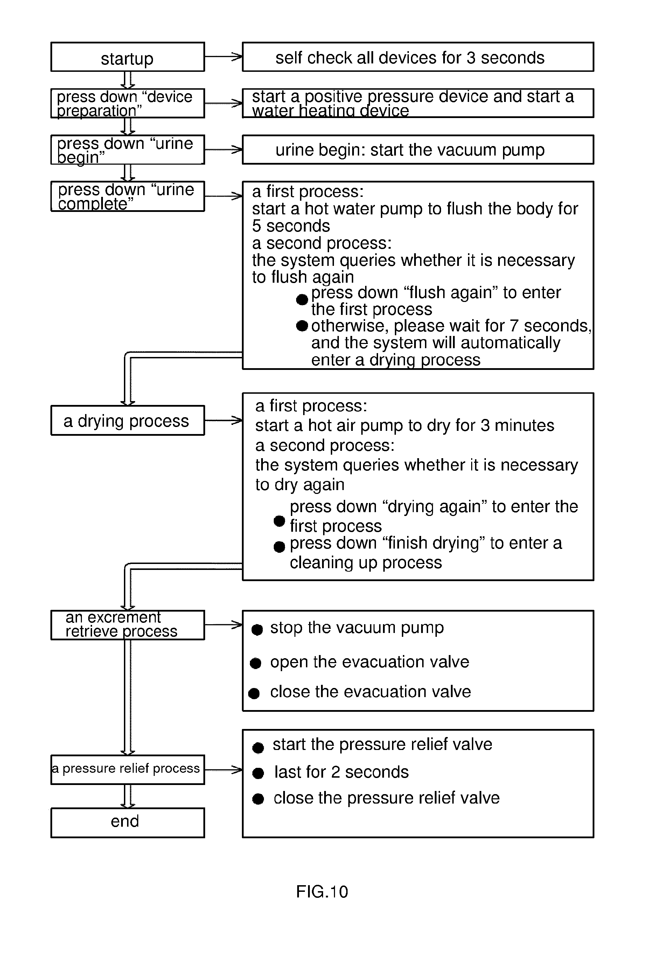

In this way, the excrement automatic cleaning apparatus without need of undressing and dressing of the present invention performs the following procedures in a sequential and cycling manner under control of the control device 6. According to the saving principle, the procedures are mainly divided into two types: an excrement and urine disposal which requires flushing and a urine disposal which does not require flushing. In the excrement and urine disposal which requires flushing, as shown in Fig. 9, first of all the apparatus is initiated, and self checks all devices for 3 seconds; the interactive unit 64 is manipulated by pressing down "device preparation" to start the positive pressure pump of the positive pressure device 7 and the water heating device 32 of the hot water device 3; by pressing down "excrement begin" to open the vacuum pump of the negative pressure device 2, so that the excrement is drawn into the evacuation barrel 1 via the excrement and urine discharging duct 92 and the inlet pipe 13 under effect of the positive pressure device 7 and the negative pressure device 2; once the excretion is complete, by pressing down "excrement complete" to open the hot water pump 33 of the hot water device 3 for flushing the body for 5 seconds; in case it is necessary to flush again, by pressing down the related switches, and otherwise, after waiting for 7 seconds, the system automatically enters a drying process. In the drying process, the hot air pump 42 in the hot air device 4 is started to dry for 3 minutes. In case that it is necessary to dry again, a "drying again" button is pressed down. Once drying is complete, the system automatically performs the excrement retrieve process. That is, the vacuum pump of the negative pressure device 2 is stopped and the evacuation barrel control valve 18 of the evacuation barrel 1 is started to flush the evacuation barrel 1, so that the excrement and waste water together enter the packing bag 51, and finally the evacuation barrel control valve 18 is stopped. Finally, in the pressure relief process, the pressure relief valve of the positive pressure device 7 is started and lasted for 2 seconds, and then the pressure relief valve is closed, thus completing the excrement procedure. As shown in Fig. 10, as compared with the excrement and urine disposal which requires flushing, the urine disposal which does not require flushing only differs in that the time for flushing of the hot water pump 33 after "urine complete" is reduced, and the flushing step in the "excrement retrieve process" is omitted. The remaining steps in this procedure are identical to those in the excrement and urine disposal which requires flushing, and thus are not described in detail herein for simplicity.

In the present invention, after the user completes excrement, the caregiver only needs to open the housing, tighten the tightening belt 512 of the packing bag 51, disassemble the mouth 510 and put it into a dustbin, and does not have to directly contact the excrement and urine. Therefore, the present invention can largely improve working conditions of the caregiver during cleaning up the evacuation barrel 1 and facilitate carrying out the nursing services. Besides, during drying with hot air, moisture produced in the self-adapting pants 9 is drawn by the negative pressure device 2 into the evacuation barrel 1, which decreases the time for drying. In addition, the moisture is substantially not removed through the human body, and this provides the passage for discharging the moisture, thus saving energy and increasing the comfort for the human body. In addition, the final pressure relief action of the positive pressure device 7 improves the comfort of wearing and usage for the user. Thus the present invention has the advantages that there is no need of undressing and dressing, convenient, simple and comfortable to use.

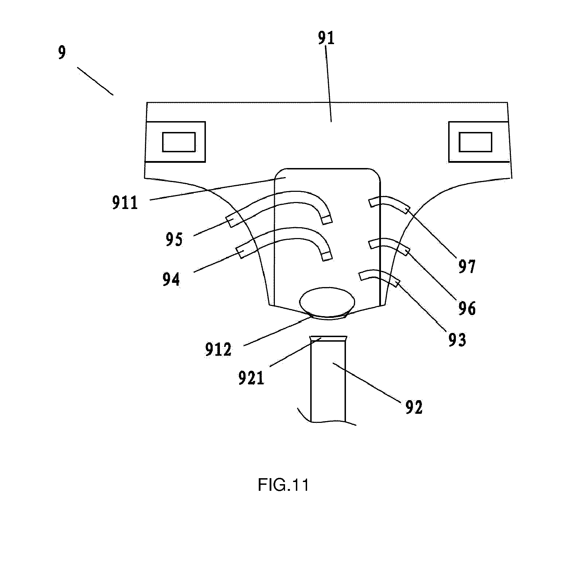

As shown in Fig. 11, the main part of the self-adapting pants 9 is a pant body 91. The facing material of the pant body 91 may be made of ordinary facing materials, like cotton, Terylene, nylon, spandex, Lycra, silk, Modal or the like. In this embodiment, the pant body 91 is movable pants which have a structure similar with diaper. A front piece and a rear piece are provided with respective sticking parts on both sides. The front piece and the rear piece may be stuck to form a triangular or straight angle pant body 91.

The crotch of the pant body 91 near the excrement and urine discharging port is covered with a waterproof soft facing layer (e.g. a waterproof nanometer facing material, or the waterproof and air-permeable film SUPER-TEX developed by GUFC in German, a silicone sheet, or a latex sheet), which constitutes a waterproof excrement collecting area 911. The excrement collecting area 911 is provided at the bottom with an excrement and urine discharging port which is provided with a pipe connector 912 made of a soft material like silicone or latex. The pipe connector 912 is very soft, comfortable and less sensible when being contact with the human body. The pipe connector substantially has no effect on the human body. The pipe connector may be formed in a shape of circle, ellipse, polygon, or the like.

The excrement and urine discharging duct 92 is a thick hose made of a soft material like silicone, latex, or the like. The thick hose may be connected with the self-adapting pants 9 for a long time, and is soft, comfortable and less sensible when being contact with the human body. The thick hose substantially has no effect on the human body. The inlet end of the excrement and urine discharging duct 92 is provided with another quick connector 921 corresponding to the pipe connector 912 at the excrement and urine discharging port. The pipe connector 912 may be quickly fastened with the quick connector 921, so that the excrement and urine discharging port communicates with the evacuation barrel 1 of the fully automatic disposal apparatus 100 via the excrement and urine discharging duct 92.

The pant body 91 is further provided with an inflating pipe 93, a hot water inlet pipe (including a urine spray pipe 94 and an excrement spray pipe 95), and a hot air inlet pipe (including a urine hot air pipe 96 and an excrement hot air pipe 97). The inflating pipe 93, the urine spray pipe 94, the excrement spray pipe 95, the urine hot air pipe 96 and the excrement hot air pipe 97 are thin hoses made of a soft material like silicone, latex or the like.

An end of the inflating pipe 93 is located in the pant body 91, and the other end is formed by extending the nozzle of the pant body 91 to the outside of the pant body 91 and is connected with the air outlet of the positive pressure device 7 via a pipe. During use, the pant body 91 is inflated by the inflating pipe 93, so that the excrement collecting area 911 is expanded and falls to form a discharging space for excrement and urine.

The outlet ends of the urine spray pipe 94, the excrement spray pipe 95, the urine hot air pipe 96 and the excrement hot air pipe 97 extend to respective positions in the pant body 91 (corresponding to the position of the excrement port or corresponding to the position of the urine excrement port). The inlet ends of the urine spray pipe 94 and the excrement spray pipe 95 are formed by extending from the nozzle of the pant body 91 to the outside of the pant body 91 and are connected with the hot water device 3 via a pipe. The inlet ends of the urine hot air pipe 96 and the excrement hot air pipe 97 are formed by extending from the nozzle of the pant body 91 to the outside of the pant body 91 and are connected with the hot air device 4 via a pipe.

The inflating pipe 93, the urine spray pipe 94 and the urine hot air pipe 96 are fixedly arranged in the front piece of the pant body 91. The excrement spray pipe 95 and the excrement hot air pipe 97 are fixedly arranged in the rear piece of the pant body 91. It is also possible to arrange interlayers in the front piece and the rear piece of the pant body 91, and then arrange the inflating pipe 93, the urine spray pipe 94, the excrement spray pipe 95, the urine hot air pipe 96 and the excrement hot air pipe 97 in respective interlayers.

It is noted that various pipes in the present invention can be formed by silicone, latex, or the like, and a quick connector may be further arranged between the pipe and the self-adapting pants 9 to improve convenience during assembly and disassembly.

The embodiments described above and the accompanying drawings do not intend to limit the form and style of the product according to the present invention, and the skilled in the art can make appropriate changes or modifications to the present invention without departing from the scope thereof.

## Claims

1. An excrement automatic cleaning apparatus without need of undressing and dressing comprises a fully automatic disposal apparatus and a toilet;
- the toilet is provided with an excrement collecting area, an excrement and urine discharging port, a hot water inlet pipe and a hot air inlet pipe, which cooperate with the fully automatic disposal apparatus;
- the fully automatic disposal apparatus comprises a housing, and an evacuation barrel, a negative pressure device, a hot water device, a hot air device and a control device in the housing, the evacuation barrel is connected with the excrement and urine discharging port of the toilet via the excrement and urine discharging duct, the control device is connected with the negative pressure device, the hot water device and the hot air device, respectively, the negative pressure device, the hot water device and the hot air device are connected with the evacuation barrel, the hot water inlet pipe and the hot air inlet pipe, respectively;
**characterized in that**,
- the fully automatic disposal apparatus is further provided with a packing device which is connected with the evacuation barrel and can seal and pack the excrement, urine and waste water;
- the toilet is self-adapting pants made of a soft facing material, the excrement collecting area is arranged at the crotch of the self-adapting pants and made of a soft and water-proof material; and
- the fully automatic disposal apparatus is further provided with a positive pressure device which can inflate the self-adapting pants so that the excrement collecting area of the self-adapting pants is expanded to form a discharging space.

2. The excrement automatic cleaning apparatus without need of undressing and dressing according to claim 1, **characterized in that**,
- the housing comprises a rear chamber and a front chamber which are arranged in a high/low manner;
- the evacuation barrel, the packing device and the control device are arranged in the rear chamber, the evacuation barrel and the packing device are arranged in an up/down manner, and the control device is arranged at a side of the evacuation barrel;
- the front chamber contains a negative pressure device, a hot air device and a hot water device which are arranged sequentially from left to right; and
- the outlets of an inlet pipe, a hot air output pipe and a hot water output pipe, which correspond to the evacuation barrel, the hot air device and the hot water device, extend from the same neighboring position of the housing.

3. The excrement automatic cleaning apparatus without need of undressing and dressing according to claim 1, **characterized in that**,
- the evacuation barrel is provided with an inlet pipe, a negative pressure pipe and an outlet pipe for communicating with the excrement and urine discharging duct, the negative pressure device and the packing device; and
- the outlet pipe is arranged at the bottom of the evacuation barrel, the outlets of the inlet pipe and the negative pressure pipe are arranged in a staggered manner, and the outlet of the negative pressure pipe is higher than the outlet of the inlet pipe.

4. The excrement automatic cleaning apparatus without need of undressing and dressing according to claim 3, **characterized in that**,
- the evacuation barrel is further provided with an evacuation barrel control valve at the outlet pipe, and the evacuation barrel control valve is connected with the control device.

5. The excrement automatic cleaning apparatus without need of undressing and dressing according to claim 3, **characterized in that**,
- the evacuation barrel is provided with a hot water pipe for communicating with the hot water device, and the outlet of the hot water pipe is located above the inlet pipe.

6. The excrement automatic cleaning apparatus without need of undressing and dressing according to claim 1, **characterized in that**,
- the packing device comprises a packing bag and a packing bag accommodating case for accommodating the packing bag, the packing bag accommodating case is located below the evacuation barrel, the packing bag is provided with a mouth for connecting with the evacuation barrel, and the mouth is provided with a tightening belt for sealing said mouth.

7. The excrement automatic cleaning apparatus without need of undressing and dressing according to claim 1, **characterized in that**,
- the hot water device comprises a water tank and a water heating device which are arranged in an up/down manner, the water tank is provided with a water inlet for feeding water at the upper portion, and the water tank is provided with supporting bars for supporting the water tank at the bottom.

8. The excrement automatic cleaning apparatus without need of undressing and dressing according to claim 1, **characterized in that**,
- the control device comprises a primary controller, a data acquisition module connected with an input terminal of the primary controller, and a driver output module connected with an output terminal of the primary controller.

9. The excrement automatic cleaning apparatus without need of undressing and dressing according to claim 8, **characterized in that**,
- the control device comprises an interactive unit connected with the primary controller, which comprises a liquid crystal display module and a key input module.

10. The excrement automatic cleaning apparatus without need of undressing and dressing according to claim 1, **characterized in that**,
- the housing is provided with road wheels for facilitating the housing to move around at the bottom.
